# FASCICULE DE BREVET EUROPEEN

(11) **EP 2 249 892 B1**
(45) Date de publication et mention de la délivrance du brevet: **15.11.2017**
(21) Numéro de dépôt: 09719739.6
(22) Date de dépôt: 20.02.2009
(51) Int. Cl.: A61L 31/10, A61L 31/16

(54) **DISPOSITIF MEDICAL IMPLANTABLE AVEC UNE COUCHE DE PROTECTION/RETENTION D'UN AGENT ACTIF OU MEDICAMENT, NOTAMMENT HYDROSOLUBLE**
IMPLANTIERBARE MEDIZINISCHE VORRICHTUNG MIT EINER SCHUTZ-/RÜCKHALTESCHICHT FÜR (INSBESONDERE LÖSLICHE) WIRKSTOFFE ODER ARZNEIMITTEL
IMPLANTABLE MEDICAL DEVICE INCLUDING A PROTECTION/RETAINING LAYER FOR AN ACTIVE INGREDIENT OR DRUG, IN PARTICULAR A WATER-SOLUBLE ONE

(30) Priorité: 21.02.2008 FR 0851136; 21.02.2008 FR 0851135; 21.02.2008 FR 0851137; 21.02.2008 FR 0851138
(43) Date de publication de la demande: 17.11.2010
(73) Titulaire: Hexacath, 92500 Rueil Malmaison (FR)
(72) Inventeur: ASCHER, Gilles, F-92200 Neuilly-sur-seine (FR); CAMENZIND, Edoardo, CH-6900 Bellinzona (CH)
(74) Mandataire: Portal, Gérard
(86) Numéro de dépôt international: PCT/FR2009/050277
(87) Numéro de publication internationale: WO 2009/112741

(56) Documents cités:
- WO-A-01/87372
- WO-A-2006/102359
- US-A1- 2003 028 243
- US-B1- 6 702 850
- DEVESH KTHWALA, ANKUR RAVAL, ANIMESH CHOUBEY, CHHAYA ENGINEER AND HARESH KOTADIA: "Paclitaxel Drug Delivery from Cardiovascular Stent" TRENDS IN BIOMATERIALS & ARTIFICIAL ORGANS, vol. 19, no. 2, janvier 2006 (2006-01), pages 88-92, XP002548439

## Description

L'invention concerne un dispositif médical implantable avec une couche de protection/rétention d'un agent actif ou médicament, notamment hydrosoluble.

Plus précisément ce dispositif médical implantable comprend un agent actif ou médicament notamment hydrosoluble, et est caractérisé en ce qu'il comprend des moyens de réception dudit agent actif ou médicament, sous forme de dépôt solide, le dispositif médical implantable comprend une surface interne et une surface externe, et lesdits moyens pour recevoir l'agent actif ou médicament sont localisés au moins en partie sur au moins une partie de la surface externe qui comprend une sculpture de surface comprenant la formation de réservoirs définissant un volume de réservoir individuel prédéterminé et permettant d'embarquer un volume ou une masse prédéterminé(e) d'agent(s) actif(s) ou médicament(s) ; et au moins une couche de protection et/ou de rétention permettant de réaliser la protection/rétention de l'agent actif ou médicament jusqu'à son site d'implantation, ladite couche comprenant un agent filmogène biocompatible et biodégradable; et au moins un agent hydrophobe, de préférence biocompatible, permettant de contrôler la vitesse de dégradabilité de la couche de protection ou de rétention.

On entend dans le cadre de la présente invention dans la description et les revendications la signification des termes suivants:
- Agent actif ou thérapeutiquement actif : tout agent ou produit ou substance ou composition, pris seul ou en combinaison avec un autre agent, produit ou substance, qui a une activité thérapeutique pouvant prévenir ou ralentir ou traiter une maladie d'êtres vivants, en particulier d'êtres humains et d'animaux. Il pourra être utilisé notamment pour la prévention ou la réparation d'un dommage causé à un tissu mou ou à un tissu osseux, pour réaliser la régulation de la cicatrisation d'une blessure, pour prévenir ou minimiser le risque de resténose, pour inhiber ou au contraire favoriser une angionénèse.
- Un médicament est ici utilisé indifféremment pour avoir la même signification et est soit un agent soit un produit ou encore une substance, qui seul ou pris en combinaison avec un autre agent, produit ou substance, a une activité thérapeutique pour prévenir ou ralentir ou traiter une maladie ou être une composition comprenant au moins un des agents thérapeutiquement actifs, habituellement avec un excipient pharmaceutiquement acceptable.

- Principe actif ou médicament hydrosoluble : on entend un principe actif ou un médicament qui est soluble en milieu aqueux et en milieu aqueux assimilé tel que milieu sanguin ou plasma.
- Stent: prothèse, placée à l'intérieur d'une cavité anatomique (endo-prothèse) afin de préserver la lumière interne,se présentant sous la forme d'un tube muni d'ouvertures dans sa paroi de différents types de formes et tailles ou d'un treillis généralement réalisé en un matériau métallique ou en une autre substance à déformation plastique, expansible ou auto-expansible.
- Agent filmogène : un agent capable de former un film;
- Agent filmogène biocompatible et biodégradable: un agent filmogène capable de former un film qui d'une part, ne génère pas de réactions tissulaires et d'autre part, qui puisse être résorbé et éliminé progressivement par l'organisme;
- Agent hydrophobe : un agent faiblement soluble en milieu aqueux, cet agent hydrophobe permet ainsi de régler le degré de solubilité d'un film ou d'une couche biocompatible et biodégradable en milieu aqueux et notamment dans le sang ou le plasma. Dans le cadre de la présente invention, l'agent hydrophobe permet de contrôler la vitesse de désagrégation de la couche de protection ou de rétention.
- Agent hydrophobe biocompatible signifie que l'agent hydrophobe ne génère pas de réactions tissulaires.
- On entend par biodégradable dans le cadre de la présente invention, le fait que la couche de protection/rétention disparaît au site d'implantation quel qu'en soit le mécanisme d'action.

L'invention assure une meilleure protection du principe actif ou médicament jusqu'à sa destination prévue.

### ETAT DE LA TECHNIQUE

### Principes actifs et moyens de délivrance

Il est connu par le document US 5, 874, 419 des compositions comprenant un dérivé polyionique de cyclodextrine combiné avec un facteur de croissance, de préférence un facteur de croissance liant l'héparine. Cette composition peut être utilisée dans une méthode pour inhiber la resténose selon diverses voies d'administration dont l'une comprend la diffusion d'une suspension aqueuse ou dispersion du dérivé de saccharide directement dans la paroi artérielle au site. Cette solution présente l'inconvénient majeur d'une diffusion de suspension aqueuse de dispersion du dérivé de saccharide par le cathéter à ballonnet d'infusion modifié, ce qui implique nécessairement une diffusion limitée dans le temps et avec une administration quasi instantanée du principe actif.

Il est encore connu par le document WO 94/22885 l'utilisation de saccharose octasulfate de sodium pour réaliser un traitement anti-trombotique, voir page 3 ligne 13.

Il est encore connu par le document US 4, 912, 093 l'utilisation du même saccharose octasulfate de sodium pour réaliser un traitement de cicatrisation de la peau ou du tissu osseux en l'utilisant en phase ou état liquide (revendications 6, 8, 9 à 11), ou encapsulé dans un polymère ou autre, voir la revendication 18 de ce document.

Il est aussi connu par l'article de Mr. CAMENZIND et al. dans Journal of Cardiovascular Pharmacology, volume 43, N°1, de janvier 2004, la délivrance intra coronaire localisée d'octréotide chez les humains sous forme d'une étude pharmacocinétique pour déterminer l'efficacité selon la dose en prévention de resténose, pages 133 à 138. La délivrance de l'octréotide est réalisée sous forme de solution saline contenant de l'octréotide radio marqué à ¹¹¹In (indium 111) 0,02 µg par millilitre infusé à un taux de 18 ml par heure pour aboutir une dose cible de 0,18 µg en 30 minutes au site de l'angioplastie, à l'aide d'un cathéter d'infusion et infusé concomitamment à de l'héparine, à raison de 200 UI/ml. A la fin de l'article, Mr. CAMENZIND envisage une délivrance par stent, mais sans donner aucun moyen technique pour y aboutir.

Il est également connu, par le document US 2005/0239743 A1, des compositions et des méthodes pour inhiber la resténose comprenant l'administration d'une composition de prévention de la resténose comprenant un ou plusieurs polysaccharides en combinaison avec un ou plusieurs agents pharmaceutiquement actifs parmi lesquels il est cité l'octréotide (voir revendications 1 et 5).

Il est prévu dans ce document aussi une possibilité de délivrer la composition à l'aide d'un dispositif d'angioplastie tel qu'un stent cardiaque, sous forme d'un revêtement (Page 1, § [0012]), mais la description ne donne aucun moyen technique sur une telle combinaison avec un stent autre que l'incorporation des composés actifs dans des polysaccharides comme les galactomananes, l'arabinogalactane, le rhamnogalacturonane, le carraghenane, et la gomme de caroube (page 1, § [0013]). Des essais in vitro sont réalisés sur la composition comprenant une combinaison de polysaccharide(s) et de paclitaxel pour tester l'inhibition de la migration ou de la prolifération de cellules musculaires lisses ("Smooth Muscular Cells").

Egalement, le document US 7 084 117 décrit des compositions pharmaceutiques, qui inhibent la prolifération vasculaire ainsi qu'une méthode d'utilisation de celle-ci. Ce document prévoit l'emploi de substances agonistes de récepteurs de type 1 de somatostatine, qui peuvent être disposées sur un stent vasculaire (revendication 10). Il est indiqué que cet agoniste dit SSTR-1 serait typiquement appliqué au moment de la chirurgie de préférence sous forme d'une formulation à libération contrôlée et/ou d'une technologie réalisant une barrière.

Encore, le document EP 1 470 830 de MEDTRONIC VASCULAR décrit un stent revêtu d'un médicament lié dans un polymère comprenant un copolymère de polysulfone et de styrène sous forme séquencée (block).

Il est aussi connu par le document US 5,861,168 une méthode pour réduire la probabilité de resténose résultant d'une lésion vasculaire comprenant un agent précurseur de NO tel que la L-arginine ou la L-lysine sous forme de solution aqueuse pouvant être délivrée par un cathéter (revendications 1, 3 et 4).

Il est encore connu par le brevet US 6,994,867 une composition pour inhiber la resténose qui comprend un oligomère de L-arginine , la L-arginine ou un analogue, lié par une liaison labile à une matrice de polymères (voir les revendications).

Le brevet US 2003/028243, inventeur BATES, société COOK, décrit une couche externe poreuse 20 biocompatible dont la porosité est réglée pour permettre une libération contrôlée du principe actif situé dans une couche sous-jacente 18 (voir en particulier paragraphe [0058], page 6).

Le brevet WO/0187372 de CORDIS vise des combinaisons de médicaments utiles pour la prévention de la resténose sur la base d'une thérapie multi-médicament combinée à un stent (voir page 9). Ce document prévoit en référence aux figures 1 à 4 de modifier la surface des stents pour fournir une délivrance adéquate de médicaments. Dans le cadre du tableau 2, dans l'exemple 1, il est présenté la présence de médicament comélangé avec un polymère. L'exemple 2 montre un exemple de multicouches dans lequel le stent est tout d'abord revêtu d'une couche de parylène C™. Au tableau 2, certains exemples montrent une couche de base comprenant de la rapamycine mélangée au polymère et une couche supérieure de dexaméthasone mélangée au polymère ou inversement, ou même le mélange des médicaments dans le polymère et ensuite une couche barrière à base de polybutyleméthacrylate (voir les deux derniers exemples du tableau 2 page 13, vers la ligne 4).

Le document WO2006/102359 est relatif à un dispositif médical qui peut comprendre des réservoirs tels que 10. Ce document prévoit la libération d'un certain nombre de principes actifs, qui sont listés au tableau 1 en page 33. Il est précisé en page 35 qu'il peut être prévu avec l'agent pharmaceutique une quantité efficace d'un inhibiteur d'hydratation pour réduire le caractère hydrophile de l'agent bénéfique afin de contrôler sa délivrance (page 35, 1er paragraphe complet). Cet inhibiteur d'hydratation peut être un matériau polymère (page 35, dernière ligne). Des exemples multiples sont donnés (page 36, lignes 2 à 10). Comme exemple non limitatifs d'agents bénéfiques utiles comme inhibiteur d'hydratation, il est aussi cité divers principes actifs, et en particulier la rapamicyne et ses dérivés, le phénofibrate, les taxotères, le zotarolimus (voir page 36, dernier paragraphe et page 37). Toute une liste de matériaux polymères est ensuite donnée en page 37 jusqu'en page 41. L'exemple 1 concerne des expériences avec des coupons avec un chargement en médicament et ensuite un essai d'extraction du médicament à partir du coupon (voir pages 52 à 59). L'exemple 3 vise la protection de la dexaméthasone par la présence de zotarolimus. La dexaméthasone est utilisée comme médicament.

Le brevet US 6 702 850 B1 de la société MEDIPLEX vise un stent ayant un revêtement multicouche adhérant à sa surface, qui peut prévenir de la resténose et la trombose au site d'implantation. Le stent comprend au moins deux couches de revêtement. La première couche est un polymère avec un ou plusieurs agents actifs biologiquement dispersé(s) dans celui-ci. La seconde couche est formée par un polymère à base d'héparine hydrophobe, qui inhibe la coagulation du sang et forme une surface hydrophobe avec réduction de la friction entre le stent et le site où se trouve la lésion (voir l'abrégé et les figures). Il est précisé en colonne 3, lignes 30 à 43 que le polymère antithrombotique à l'héparine est préparé par la liaison des macromolécules multifonctionelles, telles que l'acide polyacrylique et des matériaux hydrophobes, tels que l'octadécylamine avec l'héparine. Il est encore précisé que ce polymère héparinisé antithrombotique réduit ou évite la libération brutale de l'agent biologiquement actif de la première couche, en permettant ainsi une libération de ce produit sur une période de temps relativement prolongée (colonne 3, lignes 53 à 57).

### BUTS DE L'INVENTION

La présente invention a pour but principal de résoudre le nouveau problème technique consistant en la fourniture d'une solution technique qui permette d'administrer un agent thérapeutiquement actif ou un médicament notamment hydrosoluble, sous forme de dépôt solide, tout en étant protégé sur son parcours jusqu'au site d'administration par une couche de protection/rétention dont, selon une réalisation particulière, la désagrégation peut être avantageusement contrôlée sur une période de temps donnée.

La présente invention a encore pour but principal de résoudre ce nouveau problème technique selon une solution technique qui permette l'administration de cet agent thérapeutiquement actif ou de ce médicament selon une voie d'administration habituelle, avec si possible une solution permettant de déterminer la quantité et/ou la période de temps de libération de l'agent actif ou médicament, et notamment compatible avec l'emploi d'un dispositif médical implantable, en particulier un stent.

La présente invention a encore pour but principal de résoudre les nouveaux problèmes techniques précités, selon une solution technique qui permette de réaliser un traitement de la resténose, et notamment de la resténose vasculaire, et en particulier au niveau cardiaque.

L'ensemble de ces problèmes techniques est résolu pour la première fois par la présente invention, d'une manière sûre et fiable, utilisable à l'échelle industrielle et médicale.

### DESCRIPTION DETAILLEE DE L'INVENTION

L'ensemble des problèmes techniques énoncés ci-dessus est résolu pour la première fois par la présente invention d'une manière simple, sûr et fiable, utilisable à l'échelle industrielle et médicale.

Selon un premier aspect, l'invention concerne un dispositif médical implantable comprenant au moins un agent thérapeutiquement actif ou médicament notamment hydrosoluble, caractérisé en ce qu'il comprend des moyens de réception dudit agent thérapeutiquement actif ou médicament, sous forme de dépôt solide, le dispositif médical implantable comprend une surface interne et une surface externe, et lesdits moyens pour recevoir l'agent actif ou médicament sont localisés au moins en partie sur au moins une partie de la surface externe qui comprend une sculpture de surface comprenant la formation de réservoirs définissant un volume de réservoir individuel prédéterminé et permettant d'embarquer un volume ou une masse prédéterminé(e) d'agent(s) actif(s) ou médicament(s) ; et au moins une couche de protection/rétention biocompatible et biodégradable permettant de réaliser la protection de l'agent thérapeutiquement actif ou médicament jusqu'à son lieu implanté, ladite couche de protection comprenant au moins un agent filmogène biocompatible et biodégradable, et au moins un agent hydrophobe, de préférence biocompatible, permettant de contrôler la vitesse de désagrégation de la couche de protection/rétention.

On entend par l'expression "sous forme de dépôt solide" que l'agent ou le médicament est déposé sous forme de dépôt ou phase solide sur ou dans les moyens de réception, en particulier pour former au moins une couche solide dudit agent ou médicament.

Selon une autre caractéristique optionnelle, l'agent filmogène biocompatible et biodégradable est choisi parmi un polyalkylèneglycol, une polyvinylpyrrolidone, ou leurs mélanges en toutes proportions.

Selon encore une autre caractéristique optionnelle, le polyalkylèneglycol comprend ou est constitué de polyéthylèneglycol.

Selon une caractéristique optionnelle, l'agent hydrophobe biocompatible comprend la dexaméthasone ou un dérivé de la dexaméthasone.

Selon encore une autre caractéristique particulière, le dérivé de dexaméthasone comprend la dexaméthasone, la dexaméthasone phosphate, la dexaméthasone acétate.

Selon une autre caractéristique optionnelle, le rapport relatif entre l'agent filmogène biocompatible et biodégradable et l'agent hydrophobe varie de 99 % à 1 % d'agent filmogène pour 1 % à 99 % en poids d'agent hydrophobe.

Selon encore une autre caractéristique optionnelle, la couche de protection/rétention déposée sur le principe actif ou médicament lui-même préalablement déposé ou chargé sur les moyens de réception précités comprend de 0.1 à 100 µg de composant dexaméthasone par mm² de surface de couche de protection/rétention.

Selon une caractéristique particulière, lesdites formations de réservoir peuvent être choisies parmi le groupe comprenant des incisions, des canaux, des rainures, des puits ou cavités.

Selon une autre caractéristique particulière, lesdites formations de réservoir ont un fond fermé concave ou non-concave.

Selon un mode de réalisation particulier, le dispositif médical comprend un stent ayant une surface externe et une surface interne, lesdits moyens pour recevoir ledit agent actif ou médicament sont au moins en partie localisés sur au moins une partie de ladite surface externe et comprennent une sculpture de surface du stent.

Un exemple d'un dispositif médical implantable, qui peut être un stent, qui comprend une sculpture de surface, est divulgué dans le document EP 1 180 903 B1 et auquel l'homme de l'art peut prendre toute information utile. Ce document montre la réalisation de la formation de réservoirs sur la surface du dispositif médical implantable tel qu'un stent, qui peut être sous la forme d'incisions, sous la forme de canaux ou de rainures, et qui peuvent avoir un périmètre fermé sur les parois latérales et un sommet ouvert pour fournir des réservoirs pour l'agent actif ou médicament appliqué sélectivement sur les formations. Toutes les formations décrites ainsi que la méthode de formation peuvent être utilisées dans le cadre de la présente invention.

Selon un mode de réalisation particulier, il peut être utilisé dans le cadre de l'invention tout dispositif médical implantable notamment tout stent de l'art antérieur, et notamment ceux décrits dans EP 1 180 903 ou dans EP 1 277 449. Ce dernier document montre la prévision d'évidements réalisés sur la surface interne du stent.

Un autre exemple d'un procédé pour la production de stent pour angioplastie avec une sculpture de surface est décrit dans EP 0 850 604 B1. Selon un autre mode de réalisation particulier de l'invention, il peut être utilisé un dispositif médical implantable comme un stent réalisé en acier inoxydable ou réalisé à partir d'une composition en alliage, tel que décrit dans le document antérieur du déposant Hexacath EP-1 674 117. En particulier, le stent peut avoir une couche additionnelle passive ou bien inerte comme décrit dans ce brevet.

Selon une autre caractéristique particulière, le dispositif médical implantable, tel qu'un stent, peut être pourvu d'une sculpture de surface plus spécifique, comprenant la formation sur la surface externe du dispositif médical implantable au moins en partie de microréservoirs, tels que des micropuits ou microcavités, ayant dans un mode de réalisation particulier une forme hémisphérique, ou ovoïde, ou un contour ellipsoïdal, dans lequel le fond des microréservoirs peut être de forme quelconque et notamment de forme fermée concave ou non concave.

Il est à noter que la taille, la forme et le nombre des microréservoirs, tels que micropuits ou microcavités, détermine d'une part, la quantité d'agent actif ou médicament embarqué et d'autre part, la période de temps de libération de celui-ci.

Selon une caractéristique particulière, le volume du réseau fourni par les micros réservoirs, c'est-à-dire le volume de chaque micro réservoirs, en conséquence le volume total fourni, peut être contrôlé, prédéterminé et défini par trois paramètres, qui peuvent être utilisé individuellement ou en combinaison.

Les paramètres de contrôle de volumes comprennent :
a) la taille individuelle des micros réservoirs qui peuvent être de taille micrométrique, notamment dans le domaine compris entre environ 1 µm et environ 600 µm, en particulier entre 10 et 150 µm, en taille moyenne.
b) la profondeur et la forme du fond fermé des micros réservoirs la profondeur étant contrôlée pour éviter de favoriser des faiblesses mécaniques et des ruptures ou fractures ou fissures potentielles dans le dispositif médical, notamment sous la forme d'un stent.
c) le nombre total des micros réservoirs présent sur la surface externe du dispositif médical implantable, notamment un stent.

Typiquement, le nombre total des micros réservoirs peut être compris entre 1000 et plus d'un million sur un dispositif médical implantable donné.

Avec un tel volume total du réseau des micros réservoirs, tels que des micros cavités ou des micros puits, tels que définis précédemment ou dans la description qui suit prise dans son ensemble, la quantité d'un agent thérapeutiquement actif ou d'un médicament, présent sous forme d'un dépôt solide dans les cavités ou puits de la sculpture de surface et selon la présente invention, peut varier de 0,1 µg à plus de 1 g, dépendant notamment des caractéristiques physiques et chimiques de l'agent thérapeutiquement actif ou du médicament déposé.

Selon un exemple de réalisation particulier, le nombre total des micros réservoirs réalisés sur la surface apparente du stent implantable peut être compris entre 100 et 15000 sur un stent ayant une longueur d'environ 16 mm.

La méthode de fabrication des microréservoirs, tels que micropuits ou microcavités, est bien connue de l'homme de l'art et peut résulter des documents de l'art antérieur précédents révélant une sculpture de surface d'un stent. Toute nouvelle sculpture de surface est bien évidemment incluse dans le cadre de l'invention.

Selon encore une autre caractéristique optionnelle, le stent comprend un substrat et au moins une couche de revêtement céramique avec éventuellement au moins une couche d'adhésion métallique non poreuse intermédiaire comme cela est décrit dans le document antérieur du déposant EP-1 674 117.

Selon encore une autre caractéristique optionnelle, une couche de protection et/ou de rétention biocompatible et biodégradable est déposée en surface externe, sous forme de dépôt solide, dans lesdits microréservoirs, au-dessus dudit agent actif ou médicament lui-même présent préalablement sous forme ou phase solide, afin d'en assurer sa protection jusqu'à son site d'implantation, ainsi que la libération dudit agent actif ou médicament au voisinage du site implanté.

L'homme de l'art comprend que la couche de protection est suffisamment résistante à la désagrégation pendant l'implantation du dispositif médical jusqu'au site d'implantation, pour que cette couche de protection empêche essentiellement la perte du principe actif jusqu'au site d'implantation, le médicament ou agent actif déposé dans les micros réservoirs sous forme de dépôt solide restant ainsi protégé jusqu'au site d'implantation du dispositif médical. Après implantation du dispositif médical et obtention d'un contact direct du dispositif médical avec les tissus environnants, le médicament ou agent actif est localement biodisponible. Pour un stent, après déploiement dans la structure anatomique traitée, le médicament ou agent actif, présent dans les microréservoirs disposés sur la surface externe du dispositif, est localement biodisponible. La structure anatomique traitée consiste en la paroi d'un vaisseau sanguin, un tissu mou ou un tissu osseux. Quand la structure traitée consiste en une paroi d'un vaisseau sanguin, après déploiement du stent, l'accès du flux sanguin à la surface externe du stent et dans les microréservoirs contenant le principe actif ou le médicament est fortement limité.

Selon un premier mode de réalisation particulier, le médicament ou agent actif, notamment hydrosoluble, présent sous forme d'un dépôt solide, comprend un agent de promotion de la cicatrisation, sous forme d'un dépôt solide, en une quantité efficace pour induire une réponse de cicatrisation/guérison de tissus lésés au voisinage du dispositif médical implanté.

Selon une variante de réalisation particulière, ce dispositif est caractérisé en ce que l'agent de promotion de cicatrisation comprend ou consiste en un saccharide non-réducteur ou un analogue sulfaté de celui-ci.

Selon une autre variante de réalisation particulière, l'agent de promotion de cicatrisation comprend ou consiste essentiellement en un disaccharide non réducteur ou un analogue sulfaté de celui-ci.

Selon encore une variante de réalisation particulière, l'agent de promotion de cicatrisation comprend ou consiste essentiellement en saccharose ou un analogue sulfaté de celui-ci.

Selon une autre variante de réalisation particulière, l'agent de promotion de cicatrisation comprend ou consiste essentiellement en un monosaccharide ou un analogue sulfaté de celui-ci.

Selon encore une autre variante de réalisation particulière, l'agent de promotion de cicatrisation comprend ou consiste essentiellement en du glucose ou un analogue sulfaté de celui-ci.

Selon une autre variante de réalisation particulière, l'agent de promotion de cicatrisation comprend ou consiste essentiellement en du fructose ou un analogue sulfaté de celui-ci.

Selon une caractéristique particulière de l'invention, l'analogue sulfaté précité est sous la forme d'un sel choisi parmi un sel de sodium, un sel de potassium, ou leurs mélanges.

Selon un exemple de réalisation particulier, l'agent de promotion de cicatrisation comprend ou consiste essentiellement en du saccharose octasulfate de sodium.

Selon un autre mode de réalisation particulier de l'invention, complètement indépendant du mode de réalisation précédent, mais pouvant aussi être combiné avec celui-ci, le dispositif selon l'invention est caractérisé en ce que l'agent actif ou médicament, notamment hydrosoluble (36), comprend au moins un inhibiteur de facteurs de croissance, tels que les IGF, sous forme de dépôt solide.

Selon une variante de réalisation particulière de l'invention, l'inhibiteur de facteurs de croissance comprend l'octréotide, sous forme de dépôt solide.

Selon encore un autre mode de réalisation particulier de l'invention, complètement indépendant des modes de relations précédents, mais pouvant aussi être combiné avec ceux-ci, le dispositif selon l'invention est caractérisé en ce que l'agent actif ou médicament, notamment hydrosoluble (36), comprend un agent précurseur de NO, sous forme solide.

Selon une variante de réalisation particulière de l'invention, l'agent précurseur de NO est choisi parmi la L-arginine, la L-lysine, ou leurs mélanges.

Selon encore un autre mode de réalisation particulier de l'invention, le dispositif est caractérisé en ce qu'il comprend au moins un agent thérapeutique additionnel, en particulier un agent anti-resténose. Selon une variante de réalisation particulière, l'agent anti-resténose est choisi parmi un médicament inhibant la prolifération des cellules musculaires lisses, un inhibiteur du cytosquelette et un antibiotique triène macrocyclique. Cet agent anti-resténose peut être hydrosoluble ou non hydrosoluble. Comme exemple d'agent anti-resténose hydrosoluble, ce dernier peut appartenir à la famille des peptides tandis que comme médicament non hydrosoluble un exemple est un médicament appartenant à la famille des limus.

Chacun de ces agents anti-resténose est bien connu de l'homme de l'art et il n'est donc pas nécessaire de les détailler.

### Caractéristiques techniques de la couche de protection et/ou rétention :

La couche de protection et/ou rétention constitue un système de film contrôlant la protection/rétention d'agent(s) actif(s) ou médicament(s) hydrosoluble(s) dans le cadre de dispositifs médicaux implantables, parmi lesquels on peut citer des implants tels que des implants coronaires et notamment des stents, qui peuvent comprendre une sculpture de surface (microsculpture).

En outre, lors du processus d'implantation de ces dispositifs médicaux implantables, par exemple un stent, un implant ou une prothèse, et en particulier pendant la phase de montée vers le lieu d'implantation, cette couche de protection/ rétention permet de protéger et d'éviter la perte d'agent actif embarqué avec ledit dispositif implantable. Ainsi, la rétention de l'agent actif est effective jusqu'au site d'implantation et permet donc de garantir et de contrôler l'acheminement de la quantité d'agent actif ou médicament.

Selon une caractéristique particulière éventuelle, on pourra utiliser un dispositif implantable dit à microsculptures, notamment un stent à microsculptures. On entend par "microsculptures", la fabrication de microsculptures telles que des cavités, puits ou rainures en surface, notamment surface extérieure, du dispositif médical implantable, permettant d'embarquer un volume ou une masse contrôlée d'agent actif ou de médicament. La microstructure a aussi pour but de protéger l'agent actif lors de la pose du dispositif médical respectivement lors du déploiement du stent en évitant toute perte de l'agent actif avant l'apposition contre la paroi de la structure anatomique par exemple la paroi d'un vaisseau sanguin, tel qu'une artère. Un tel dispositif médical implantable peut être réalisé en divers matériaux dits "biocompatibles" bien connus de l'homme de l'art, par exemple en acier inoxydable 316L, en nitinol, qui peut être recouvert d'une ou plusieurs couches améliorant les propriétés mécaniques comme décrit dans le document antérieur du déposant EP-1 674 117.

### Les éléments constitutifs de la couche de rétention :

La couche de rétention est constituée d'un ou de plusieurs agents filmogènes biocompatibles et bio-résorbables et d'un agent hydrophobe, de préférence biocompatible, permettant de retarder la désagrégation de la couche de rétention.

### Agents filmogènes :

L'agent filmogène biocompatible et biodégradable peut être choisi parmi un polyalkylèneglycol, une polyvinylpyrrolidone, et leurs mélanges en toutes proportions.

Le polyalkylèneglycol peut être le polyéthylèneglycol, en particulier le polyéthylène glycol 8000.

L'agent filmogène biocompatible et bio-résorbable présente une concentration dans le film formant la couche de protection/rétention supérieure à 1 % en poids s'il est le seul agent filmogène constitutif de la couche de rétention.

L'utilisation de tout autre polymère biocompatible et bio-résorbable de la famille des glycols est possible de même que toute combinaison de ces polymères glycoliques pouvant donner en résultat final un copolymère.

Un polymère biocompatible et bio-résorbable tel que la PolyVinyle Pyrrolidone PVP (Numéro CAS : 9003-39-8) peut également être utilisé comme agent filmogène de la couche de protection/rétention. De plus, une combinaison d'agent filmogène à base de polymère glycolique et de PVP peut également être utilisée.

### Agents permettant de retarder la désagrégation de la couche de protection rétention :

Pour retarder la désagrégation, en particulier le délitement et/ou la dissolution, de la couche de protection/rétention de l'agent actif ou médicament, on utilisera un agent hydrophobe, de préférence biocompatible, ou un mélange d'agents dont un au moins est hydrophobe. Comme agent hydrophobe, on utilisera en particulier la dexaméthasone ou un dérivé de celle-ci, qui est très faiblement soluble en milieu aqueux. La déxaméthasone est un agent permettant de retarder de manière efficace la désagrégation de la couche de rétention.

L'agent dexaméthasone est biocompatible. En outre, tous les dérivés de la déxaméthasone ayant une hydrophobicité relative, tels que par exemple le sel disodique de déxaméthasone phosphate (N° CAS 2392-39-4) ou la déxaméthasone acétate (N° CAS 1177-87-3) peuvent être utilisés comme agent permettant de retarder la désagrégation de la couche de protection/rétention.

L'homme de l'art comprendra que les candidats éligibles comme agent permettant de retarder la désagrégation de la couche de protection/rétention doivent donner un caractère hydrophobe relatif à la couche de protection/rétention et posséder de préférence une innocuité reconnue dans ce type d'application et un caractère de préférence biocompatible prouvé de longue date.

### Mode de déposition :

Le dépôt de la couche de protection/rétention à la surface du dispositif médical implantable pourra avoir lieu par toute technique connue de l'homme de l'art, comme par exemple par vaporisation, nébulisation ou pulvérisation telle que la technique dite « air spray », par vaporisation, nébulisation ou pulvérisation ultrasonique ou par tout autre système pouvant déposer des films par nébulisation, pulvérisation de solutions.

Le dépôt de la couche de protection/rétention peut être également effectué par la technique dite de « jetting ». Cette technique dite de « jetting », bien connue de l'homme de l'art, est directement issue des techniques de déposition ou d'impression par jet d'encre ou "Ink-jet deposition".

On peut encore réaliser la couche de protection/rétention par une technique dite d'immersion ou de plongée ou en anglais technique de "dipping".

Il est inutile de décrire plus en détail toutes ces techniques car toutes ces techniques sont bien connues de l'homme de l'art.

### Composition de la Couche de Protection/Rétention :

Les caractéristiques physico-chimiques de cette couche ou de ce film contrôlent sa désagrégation dans le temps lorsque que le film est soumis à des conditions aqueuses, sériques, plasmatiques et/ou sanguines.

### • Listes des éléments constitutifs, rôles et compositions de la couche de rétention-élution :

Pour ce faire, on donne ci-après le meilleur mode actuel de réalisation de la couche de protection/rétention. Il est apparent pour l'homme de l'art que d'autres agents filmogènes ou d'autres agents permettant de retarder la vitesse de désagrégation de la couche de rétention peuvent être utilisés de manière équivalente.

| composant | Rôle principal | Rôle secondaire | Composition dans le film |
|---|---|---|---|
| PEG 8000 | Agent filmogène | agent de biodisponibilité | > 1% poids% |
| Dexaméthasone | Agent permettant de retarder la vitesse de désagrégation de la couche de rétention | Pas d'activité en raison d'une quantité trop faible | • 1 à 99%poids |
| | | | • 0,1 à 100 microg/mm² de surface de la couche de protection/ rétention |
| Eau | | Modification de la tension de surface du film déposé | 0-15% volumique |
| Méthanol | Solvants | | 40-100% volumique |
| Ethanol | | | 0-20% volumique |
| Isopropanol | | | 0-40% volumique |
| Butanol et/ou isobutanol | | | 0-40% volumique |

| | | | |
|---|---|---|---|
| • Performances, temps de tenue du film de rétention : **de 1 à 45 minutes.** • Epaisseur du film de protection/rétention : **0,1 à 20 µm.** | | | |

Selon un mode de réalisation particulier, on peut prévoir de 40 à 70% en poids d'agent hydrophobe et de 60 à 30% en poids d'agent filmogène dans la couche de protection/rétention.

### Epaisseur de la Couche de protection/rétention et le nombre possible de sous-couches :

Il est en général souhaitable que l'épaisseur totale de la couche de protection/rétention ne soit pas trop importante. En effet, une épaisseur trop importante pourrait modifier le profil et diminuer la navigabilité du dispositif médical implantable, tel qu'un stent. Il est généralement souhaitable que l'épaisseur totale de la couche de protection/rétention ne dépasse pas les 20 micromètres (microns).

Le cas le plus simple est que la couche de protection/rétention soit composée d'une unique couche d'épaisseur et de composition données. Cependant, en fonction des besoins, on peut envisager le cas où la couche de protection/rétention soit composée de plusieurs sous-couches de protection/rétention d'épaisseurs et de compostions variables. L'épaisseur totale de la couche de protection/rétention est alors la somme des épaisseurs des différentes sous-couches qui la constituent.

### Répartition et concentration en agent permettant de retarder la désagrégation de la couche de protection/ rétention :

La concentration totale en agent permettant de retarder la désagrégation de la couche de protection/rétention est fixée par la masse totale en cet agent par rapport à la masse totale de la couche de protection/rétention.

L'agent permettant de retarder la désagrégation de la couche de protection/rétention peut être réparti de manière homogène ou uniforme sur toute l'épaisseur de la couche de protection/rétention. Dans ce cas, la force de rétention ou la vitesse de désagrégation ou de disparition (diminution de l'épaisseur) de la couche de protection/rétention est constante sur toute l'épaisseur de la couche de protection/rétention.

Pour certains besoins, il est possible d'obtenir une force de protection/rétention variable en fonction de l'épaisseur de la couche de rétention. On parle d'obtenir un gradient de la force de protection/rétention. Pour réaliser ce gradient de la force de protection/rétention, la concentration de l'agent permettant de retarder la désagrégation de la couche de protection/rétention varie en fonction de l'épaisseur de la couche de protection/rétention. Ainsi, dans une couche de protection/rétention dite monocouche, un gradient de la concentration de l'agent permettant de retarder la désagrégation de la couche de protection/rétention sur toute l'épaisseur permettra d'obtenir un gradient de la force de rétention dans la dite couche.

Un résultat similaire peut être obtenu, en combinant plusieurs sous-couches de rétention de compositions et d'épaisseurs différentes. Le gradient de la force de rétention sera la résultante des forces de rétention des différentes sous-couches individuelles.

La couche de protection/rétention peut être discontinue recouvrant seulement le(s) principe(s) actif(s) ou médicament(s) préalablement présent(s) dans les microréservoirs du dispositif médical ou du stent

Ainsi, on comprend que la force de rétention de la couche de protection/rétention est réglé pour empêcher essentiellement toute perte du principe actif ou médicament pendant le trajet du dispositif médical jusqu'à son positionnement final au site d'implantation.

### Rôles des solvants :

Les solvants utilisés n'entrent pas dans la composition de la couche de protection/rétention puisqu'ils sont totalement évaporés pour obtenir la couche "sèche" dite à l'état ou phase solide, de protection/rétention. Toutefois, l'emploi d'un mélange de solvants permet de modifier pratiquement à volonté les paramètres de dépôt de la couche de protection/rétention comme l'homogénéité, la répartition, l'épaisseur, le temps de séchage...

### METHODE DE TRAITEMENT

Selon un deuxième aspect, la présente invention vise aussi une méthode de traitement d'un sujet, être humain ou animal, en ayant besoin comprenant la prévision d'un dispositif médical implantable tel que défini dans la présente description et les revendications, et l'implantation de ce dispositif médical implantable à l'endroit approprié dudit sujet, afin de réaliser le traitement approprié.

Ce traitement approprié peut être un traitement médical avec au moins un agent actif ou un médicament dont il est souhaité une action locale au site d'implantation. Cet agent actif ou ce médicament peut être utilisé pour réaliser la prévention ou la réparation d'un dommage causé à un tissu ou à un os, pour réaliser la régulation de la cicatrisation d'une blessure, pour prévenir ou minimiser le risque de resténose, pour inhiber ou au contraire favoriser une angiogenèse.

L'homme de l'art comprendra aisément que l'invention peut être utilisée pour réaliser divers traitements chez un sujet.

D'autres buts, caractéristiques et avantages de l'invention apparaîtront clairement à la lumière de la description explicative qui va suivre faisant référence à plusieurs exemples ou modes de réalisation de l'invention donnés simplement à titre d'illustration et qui ne sauraient en aucune façon limiter la portée de l'invention.

Dans les exemples, tous les pourcentages sont donnés en poids, la température est en degrés Celsius, la pression est la pression atmosphérique, sauf indication contraire.

### Description des figures

- la figure 1 représente un premier mode de réalisation d'un dispositif médical implantable, et en particulier ici un stent, ayant une surface externe comprenant une sculpture de surface ;
- la figure 2 représente une vue élargie selon la zone de la flèche II de la Figure 1, d'une partie incurvée du dispositif médical implantable, en particulier ici un stent, du dessus de la surface externe, pour mieux voir la sculpture de surface ;
- la figure 3 représente une vue encore agrandie d'une coupe transversale selon la ligne de coupe III-III de la figure 2 d'une microsculpture, ici un microréservoir 30, tel qu'un micropuit ou une microcavité avec fond fermé concave et pour montrer le dépôt solide de médicament(s) ainsi que la couche solide externe de protection/rétention, à l'intérieur de ladite microsculpture, ici un microréservoir.

### Exemples de l'invention

### Exemple 1

### Préparation d'un dispositif médical implantable, en particulier un stent, ayant une surface externe comprenant une sculpture de surface

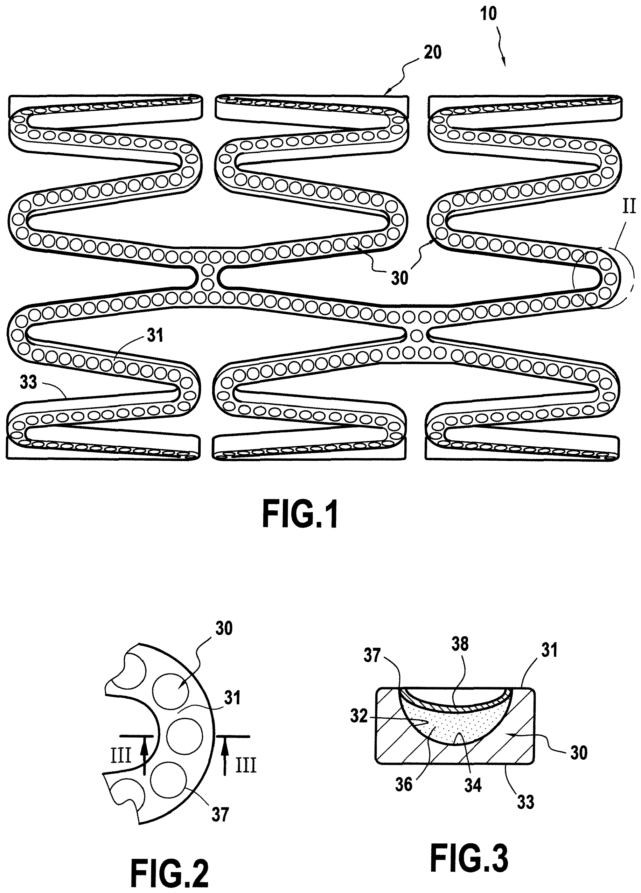

En référence à la figure 1, on a pris un dispositif médical implantable 10, par exemple ici un stent 20, réalisé ici en acier inoxydable 316L ou un acier inoxydable 316L revêtu d'au moins une couche de transition, notamment d'oxynitrure de titane, comme décrit dans le brevet précédent de HEXACATH EP-1 674 117.

Le dispositif 10, tel qu'un stent 20, comprenant une surface externe 31, destinée à venir en contact avec les tissus internes d'un sujet, en particulier un vaisseau sanguin, et une surface interne 33, a été soumis à la formation d'une sculpture de surface pour qu'il comprenne des formations de réservoirs 30 définissant un volume de réservoir prédéfini pour y déposer un agent actif ou médicament sous une phase ou à l'état solide, comme décrit ci-après à l'exemple 2.

La formation de ladite sculpture de surface a été prévue en particulier dans cet exemple sous la forme de multiples réservoirs 30 de micro taille ayant une forme de micropuits ou de microcavités avec une ou plusieurs parois latérales 32 et une paroi de fonds 34, ici de forme sensiblement hémisphérique, ainsi qu'une ouverture supérieure 37.

Le mode d'obtention de ce type de microscultures de la surface par des techniques dites de "Micro Patterning", de "Electrochemical Machining" ou de « PhotoElectrochemical Machining » est bien connu de l'homme de l'art.

Les différentes étapes qui ont permis l'obtention de ce type de microsculptures de stent de cet exemple 1 sont les suivantes :
Etape 1: La plateforme de départ est un stent, de préférence un stent de la marque Helistent® en acier inoxydable 316L de la société Hexacath, France.
Etape 2 : Préparation à la microsculpture
   Une laque de protection de type cataphorétique est déposée de manière contrôlée sur le stent.
   Par une technique d'ablation par laser, bien connue de l'homme de l'art, un réseau d'empreintes hemisphériques est créé sur la surface externe du stent sur la surface de la laque déposée.
Etape 3 : Création du réseau de microsculptures sous forme de microréservoirs tels que microcavités ou micropuits.
   Par une technique d'usinage électrochimique en milieu acide, le réseau de microsculptures sous forme de microréservoirs tels que microcavités ou micropuits est créé. La dimension de ces microréservoirs est directement liée au temps d'usinage électrochimique.
   Par exemple, un stent d'une longueur de 16 mm et plus particulièrement, un stent de la marque Helistent® de la société Hexacath pourra compter un nombre de 2900 ± 1% de microréservoirs hémisphériques d'un diamètre déterminé correspondant à un volume contrôlé souhaité.
   Dans cet exemple de réalisation, le diamètre moyen des microréservoirs hémisphériques 30 est d'environ 65 µm, la profondeur est d'environ 32 µm en moyenne; et le volume total de l'ensemble des microcavités hémisphériques est d'environ 0.2 microlitres (µl).
   On comprend que la formation de ces réservoirs permet de déposer à l'intérieur de ceux ci au moins une couche sous forme ou à l'état solide d'un ou plusieurs agent(s) actif(s) ou médicament(s), comme donné par exemple à l'Exemple 2 ci-après à titre d'illustration.

### Exemple 2

### Dépôt d'un agent actif ou médicament sous forme d'au moins une couche à l'état solide tel que de la L-arginine sur un stent à microsculptures

On a réalisé la formation d'un dépôt sous forme d'une couche à l' état solide (36) de L-arginine à l'intérieur des microsculptures ou des microréservoirs du stent tel que décrit à l'exemple 1 ci-dessus, par exemple par la mise en oeuvre des différentes étapes techniques suivantes :
Etape 1 : Nettoyage du stent à microsculptures , décrit dans l'exemple 1 de réalisation, en milieu lessiviel assisté par ultrasons avec finition air chaud ou solvant organique en phase vapeur.
Etape 2 : Activation de surface par plasma basse pression avec une atmosphère réactive oxydante. Par exemple, cette activation de la surface a été effectuée à l'aide d'une machine, modèle Femto 3 de la marque Diener Electronique disponible dans le commerce. Un exemple de conditions d'activation par plasma basse pression est un traitement de 10 minutes en plasma avec comme gaz de l'oxygène à une pression de 0.50 mbar et une puissance de 75 watts.
Etape 3, remplissage des microréservoirs en agent actif. Différentes techniques telles que le dipping (trempage ou immersion) ou le sprayage (air spray, ultrasonique spray ou autre technique de vaporisation ou nébulisation de solution) ou le "Ink-jetting" peuvent être utilisées pour effectuer spécifiquement le remplissage des microréservoirs du stent.
   Des conditions techniques satisfaisant au remplissage des microréservoirs 30, ici des microcavités, sont la pulvérisation par air-spray d'une solution aqueuse avec 15% en volume d'alcool isopropylique contenant 20% en poids de L-arginine.
   Ainsi, la solution aqueuse de L-arginine a été vaporisée sur le dispositif 10 tel qu'un stent 20, tel qu'obtenu à l'exemple 1 avec les réservoirs de micro taille 30, pour former une couche de dépôt solide 36 de l'agent actif ou médicament comprenant la L-arginine sur les surfaces internes 32-34 des réservoirs qui présentent la forme de micropuits ou de microcavités hémisphériques.
   La quantité en L-arginine déposée dans les microréservoirs 30, tels que des micropuits ou microcavités, a été bien contrôlée par le volume total de ceux-ci, ainsi que par la proportion de la L-arginine dans la solution servant au dépôt solide de celui-ci.

### Exemple 3:

### Dépôt d'un agent actif ou médicament sous forme d'une couche à l'état solide d'un agent de promotion de cicatrisation, par exemple un saccharide non réducteur, ou encore par exemple le sucrose octa sulfate de sodium, dans les microstructures ou micro réservoirs

On a réalisé la formation d'un dépôt sous forme d'une couche à l'état solide (36) d'un agent de promotion de cicatrisation, par exemple, le sucrose octasulfate de sodium, à l'intérieur des microsculptures ou des microréservoirs du stent tel que décrit à l'exemple 1 ci-dessus.

Pour cela, l'agent de promotion/cicatrisation est dissous à une proportion de 30 % en poids dans de l'eau ou en milieu aqueux contenant 10 % en volume d'alcool isopropylique. Ensuite, le dépôt solide est réalisé lui-même par les étapes techniques différentes suivantes:
Etape 1: Nettoyage des stents à microsculptures, décrits dans l'exemple 1 de réalisation, en milieu lessiviel assisté par ultrasons avec finition air chaud ou solvant organique en phase vapeur.
Etape 2: Activation de surface par plasma basse pression avec une atmosphère réactive oxydante. Par exemple, cette activation de la surface peut être effectuée à l'aide d'une machine, modèle Femto 3 de la marque Diener Electronique disponible dans le commerce. Un exemple de conditions d'activation par plasma basse pression est un traitement de 10 minutes en plasma avec comme gaz de l'oxygène à une pression de 0.50 mbar et une puissance de 75 watts.
Etape 3: remplissage des microréservoirs en agent actif ou médicament.
   Différentes techniques telles que le dipping (trempage ou immersion) ou le sprayage (air spray, ultrasonique spray ou autre technique de vaporisation ou nébulisation de solution) ou le « Ink-jetting » peuvent être utilisées pour effectuer spécifiquement le remplissage des microréservoirs du stent.
   Des conditions techniques satisfaisant au remplissage des microréservoirs, 30 sont la pulvérisation par air-spray d'une solution aqueuse avec 15% en volume d'alcool isopropylique contenant 30% en poids d'agent de promotion de la cicatrisation, par exemple le saccharose octasulfate de sodium.
   Ainsi, la solution aqueuse contenant l'agent de promotion de la cicatrisation est vaporisée sur le dispositif 10 tel qu'un stent 20, tel qu'obtenu à l'exemple 1 avec les réservoirs 30, pour former une couche de dépôt solide 36 de l'agent actif ou médicament comprenant l'agent cicatrisant sur les surfaces internes 32-34 des réservoirs qui présentent ici la forme de micropuits ou de microcavités hémisphériques.
   La quantité en agent cicatrisant déposée dans les microréservoirs 30 est bien contrôlée et défini par le volume total de ceux-ci, ainsi que par la proportion de l'agent cicatrisant dans la solution servant au dépôt solide de celui-ci.

### Exemple 4

### Dépôt d'un agent actif ou médicament sous forme d'au moins une couche à l'état solide tel que agent inhibiteur de facteurs de croissance, en particulier l'octréotide, sur un stent à microsculptures

On réalise le dépôt d'un agent actif ou médicament, sous forme ou état solide (36), comprenant un agent inhibiteur de facteurs de croissance, en particulier l'octréotide, sur un stent à microsculptures tel que décrit à l'exemple 1 ci-dessus, par exemple par la mise en oeuvre des différentes étapes techniques suivantes :
Etape 1 : Nettoyage du stent à microsculptures, décrit dans l'exemple 1 de réalisation, en milieu lessiviel assisté par ultrasons avec finition air chaud ou solvant organique en phase vapeur.
Etape 2 : Activation de surface par plasma basse pression avec une atmosphère réactive oxydante. Par exemple, cette activation de la surface peut être effectuée à l'aide d'une machine, modèle Femto 3 de la marque Diener électronique disponible dans le commerce. Un exemple de conditions d'activation par plasma basse pression est un traitement de 10 minutes en plasma avec comme gaz de l'oxygène à une pression de 0.50 mbar et une puissance de 75 watts.
Etape 3, remplissage des microréservoirs 30 en agent actif. Différentes techniques telles que le dipping (trempage ou immersion) ou le sprayage (air spray, ultrasonique spray ou autre technique de vaporisation ou nébulisation de solution) ou le « Ink-jetting » peuvent être utilisées pour effectuer spécifiquement le remplissage des microréservoirs du stent.
   Des conditions techniques satisfaisant au remplissage des réservoirs 30, ici des microréservoirs, sont la pulvérisation par air-spray d'une solution aqueuse avec 15% en volume d'alcool isopropylique contenant 10 mg/ml d'octréotide.
   Selon un premier mode de réalisation, la solution aqueuse d'un agent inhibiteur de facteurs de croissance, ici l'octréotide, est vaporisée sur le dispositif 10 tel qu'un stent 20, tel qu'obtenu à l'exemple 1 avec les réservoirs de microtaille 30, pour former un dépôt solide 36 de l'agent actif ou médicament, dans ce cas un agent inhibiteur de facteurs de croissance, ici l'octréotide, sur les surfaces internes 32-34 des réservoirs 30 qui présentent ici la forme de micropuits ou de microréservoirs hémisphériques.
   La quantité en agent inhibiteur de facteurs de croissance, en particulier l'octréotide, déposée dans les microréservoirs 30 est bien contrôlée par le volume total de ceux-ci, ainsi que par la proportion de l'agent inhibiteur de facteur de croissance, ici l'octréotide, dans la solution servant au dépôt solide de celui-ci.
   Selon un second mode de réalisation, cette solution aqueuse d'un agent inhibiteur de facteurs de croissance, ici l'octréotide est déposée sélectivement à l'intérieur des microréservoirs 30, tels qu'obtenus à l'exemple 1, du dispositif médical implantable 10, ici un stent intravasculaire 20, pour obtenir un dépôt solide 36 de l'agent actif ou médicament, dans ce cas un agent inhibiteur de facteurs de croissance, ici l'octréotide, sur les surfaces internes 32-34 des réservoirs qui présentent ici la forme de microréservoirs hémisphériques, par exemple par la technique précitée de "Ink-Jetting" ou de "jet d'encre".
   En outre, ce dépôt solide, a été obtenu, à l'aide d'un module de génération de microgouttes basé sur le principe des technologies "drop on Demand" connu de l'homme de l'art. Préférentiellement, un nombre donné de microgouttes de 60 µm (micromètres) de diamètre présentant un volume de 0.1 nL (nanolitre) ont été déposées sélectivement dans les microréservoirs 30 du dispositif médical implantable 10, ici un stent intravasculaire 20 tel qu'obtenu à l'exemple 1.

### Exemple 5: Dépôt d'une couche solide externe de protection/rétention de préférence biocompatible et biodégradable

Sur le dispositif médical implantable 10, tel qu'obtenu à l'Exemple 2, pourvu de médicament comprenant ici la L-arginine, ou tel qu'obtenu à l'Exemple 3 comprenant un agent de promotion de cicatrisation, ou encore tel qu'obtenu à l'Exemple 4 comprenant un agent inhibiteur de facteurs de croissance, en particulier l'octréotide. Il a été déposé en outre une couche externe biocompatible et biodégradable 38 de la manière suivante :
Une couche solide externe de protection/rétention 38 a été formée en préparant tout d'abord une solution à 50% en poids d'agents filmogènes comprenant le polyéthylène glycol 8000 disponible sur le marché; 50 % en poids de dexaméthasone, comme agent hydrophobe; le tout solubilisé dans un mélange de solvants comprenant par exemple 40-100 % en volume de méthanol et de 0 à 40 % en volume de butanol.

Selon un premier mode de réalisation spécifique, cette solution a été vaporisée à l'air sur le dispositif médical implantable 10, ici un stent intravasculaire 20, comprenant le médicament, la L-arginine, tel qu'obtenu à l'exemple 2 ; ou un médicament favorisant la cicatrisation tel qu'obtenu à l'Exemple 3 ; ou encore un médicament comprenant un agent inhibiteur de facteurs de croissance, en particulier l'octréotide tel qu'obtenu à l'Exemple 4, pour obtenir une couche externe solide de protection/rétention d'une épaisseur comprise entre 0,1 et 20 µm. Il a été déposé une couche de protection/rétention de composition et d'épaisseur donnée(s), par exemple par la technique précitée de nébulisation/pulvérisation.

En outre, cette couche de rétention, a été obtenue par un système standard de nébulisation/pulvérisation par air disponible dans le commerce. Préférentiellement, le modèle utilisé est un «"air- brush" BADGER, modèle 150™ (N° série WE-150-4 PK) avec des dimensions de buses M et L. Ce Badger 150™ était couplé à un compresseur à air à membrane de type WE-Atlantis modèle TF 368, 220 volts et 135 Watts.

En raison de la vaporisation essentiellement instantanée de la solution et de la très faible solubilité de l'agent actif ou médicament, dans ce cas la L-arginine ou un médicament favorisant la cicatrisation comme défini à l'exemple 3, il n'y a eu aucune désagrégation sensible du principe actif ou médicament lors de la formation de la couche de protection/rétention externe solide.

Selon un second mode de réalisation spécifique, cette solution a pu être déposée sélectivement sur les microcavités 30 comprenant le médicament précité, du dispositif médical implantable 10, ici un stent intravasculaire 20, pour obtenir une couche externe solide de protection/rétention 38 d'une épaisseur comprise entre 0,1 et 20 µm. Il a été déposé une couche de protection/rétention de composition et d'épaisseur donnée(s), par exemple par la technique précitée de "Ink-Jetting" ou de " jet d'encre".

En outre, cette couche de rétention, a été obtenue à l'aide d'un module de génération de microgouttes basé sur le principe des technologies « drop on Demand » connu de l'homme de l'art. Préférentiellement, un nombre donné de microgouttes de 60 µm (micromètres) de diamètre présentant un volume de 0.1 nL (nanolitre) ont été déposées sélectivement sur les microcavités 30 comprenant le médicament précité du dispositif médical implantable 10, ici un stent intravasculaire 20, pour obtenir une couche externe solide de protection/rétention 38 d'une épaisseur comprise entre 0,1 et 20 µm.

En raison de l'évaporation essentiellement instantanée de la solution et de la très faible solubilité de l'agent actif ou médicament, dans ce cas la L-arginine, ou le médicament favorisant la cicatrisation, tel qu'obtenu à l'Exemple 3, ou encore le médicament comprenant un agent inhibiteur de facteurs de croissance et en particulier l'octréotide, tel qu'obtenu à l'exemple 4, il n'y a eu aucune désagrégation sensible de l'agent actif ou médicament lors de la formation de la couche de protection/rétention externe solide.

### Exemple 6 Essais complémentaires

Différents exemples de Couches 38 de protection/rétention ont été réalisés. Ces différents exemples illustrent quelques variations de tenue et donc variations de la force de rétention dans des conditions in-vitro données.
Conditions In-vitro données communes à ces différents exemples :
Milieu de travail n°1 : tampon phosphate PBS à un pH de 7,4
Milieu de travail n°2 : plasma porcin à pH de 7,4
Volume du milieu de travail : 50 ml
Température du milieu de travail : 37°C ± 0,5°C
Régime de convection forcée non laminaire.

### Dépôt de la couche de protection/rétention sur un dispositif médical implantable

Sur une surface donnée, par exemple un dispositif médical implantable 10 tel qu'un stent 20 réalisé en acier inoxydable de type 316L ou acier inoxydable recouvert d'une couche de céramique biocompatible d'OxyNitrure de Titane, selon le brevet Hexacath antérieur du déposant EP-1 674 117 ou WO2006/067031, il a été déposée une couche de protection/rétention 38 de composition et d'épaisseur données, par exemple par la technique précitée de nébulisation/pulvérisation.

En outre, les différentes épaisseurs de couches de rétention, des exemples de réalisation cités ci-dessous, ont été obtenues par un système standard de nébulisation/pulvérisation par air disponible dans le commerce. Préférentiellement, le modèle utilisé est un « air- brush » BADGER, modèle 150™ (N° série WE-150-4 PK) avec des dimensions de buses M et L. Ce Badger 150™ était couplé à un compresseur à air à membrane de type WE-Atlantis modèle TF 368, 220 volts et 135 Watts.

### Essai de désagrégation de la couche de protection/rétention

Cette couche de protection/rétention a alors été immergée dans un milieu de travail et soumise à une convection forcée. Ces milieux de travail, in-vitro, soumis à une convection forcée permettent de simuler, en partie, les conditions rencontrées in-vivo lors de l'implantation des dispositifs médicaux implantables tels que des stents.

Un des paramètres permettant de mesurer la tenue et donc la force de rétention est la mesure de la vitesse de désagrégation de cette couche. La vitesse de désagrégation de la couche de rétention est directement liée à la vitesse d'apparition de la dexaméthasone dans le milieu de travail. La concentration de la dexaméthasone est donc suivie dans le milieu de travail en fonction du temps. La technique utilisée pour suivre la concentration en dexaméthasone en fonction du temps est la Chromatographie Liquide à Haute Pression ou HPLC.

### Exemple 6-1 :

| | |
|---|---|
| Milieu de travail : | n°1 et n°2 |
| Matériau de surface de l'implant (stent) : | acier inoxydable 316L et acier inoxydable 316L recouvert d'OxyNitrure de titane |
| Couche de protection/rétention : | monocouche d'épaisseur de 0,1 à 20 microns de préférence environ 12 microns |
| Composition de la couche de protection/rétention : | PEG 8000 80% Dexaméthasone 20% |
| Temps de tenue de la couche de protection/rétention : | de 1 à 45 minutes mais de préférence environ 6 minutes (toutes les configurations) |

### Exemple 6-2 :

| | |
|---|---|
| Milieu de travail : | n°1 et n°2 |
| Matériau de surface de l'implant (stent) : | acier inoxydable 316L et acier inoxydable 316L recouvert d'OxyNitrure de titane |
| Couche de protection/rétention : | monocouche d'épaisseur de 0.1 à 20 microns mais de préférence 10 microns |
| Composition de la couche de protection/rétention : | PEG 8000 67% - 33%Dexaméthasone |
| Temps de tenue de la couche de protection/rétention : | de 1 à 45 minutes mais de préférence environ 17 minutes (toutes les configurations) |

### Exemple 6-3 :

| | |
|---|---|
| Milieu de travail : | n°1 et n°2 |
| Matériau de surface de l'implant (stent) : | acier inoxydable 316L et acier inoxydable 316L recouvert d'OxyNitrure de titane |
| Couche de protection/rétention : | monocouche d'épaisseur de 0,1 à 20 microns mais de préférence 10 microns |
| Composition de la couche de protection/rétention : | PEG 8000 34% - PVP K30- 33% -Dexaméthasone 33% |
| Temps de tenue de la couche de protection/rétention : | de 1 à 45 minutes mais de préférence environ 12 minutes (Toutes les configurations) |

### Exemple 6 -4:

| | |
|---|---|
| Milieu de travail : | n°1 et n°2 |
| Matériau de surface de l'implant (stent) : | acier inoxydable 316L et acier inoxydable 316L recouvert d'OxyNitrure de titane |
| Couche de protection/rétention : | monocouche d'épaisseur de 0,1 à 20 microns mais de préférence 10 microns |
| Composition de la couche de protection/rétention : | PEG 8000 50% - Dexaméthasone 50% |
| Temps de tenue de la couche de protection/rétention : | de 1 à 45 minutes mais de préférence environ 40 minutes (Toutes les configurations) |

On comprend que grâce à l'invention, il est possible d'obtenir une couche de protection/rétention dont la vitesse de désagrégation est réglable à volonté afin de permettre une libération contrôlée de l'agent actif ou du médicament qui sera déposé en couche solide 36 sur le dispositif médical implantable 10 (ou 20) avant le dépôt de la couche de protection/rétention 38 elle même. Comme indiqué précédemment, c'est la vitesse de désagrégation de la couche de protection/rétention qui règle principalement la vitesse de libération du médicament ou principe actif déposer en couche solide 36 sur le dispositif médical implantable, en particulier dans les micros réservoirs ou micros cavités, dans la mesure où la vitesse de dissolution ou d'administration du médicament ou principe actif déposé sous forme solide dans les micros réservoirs micros cavités dépend de la capacité du flux sanguin à accéder audit dépôt solide, qui est protégé par l'application sous forte pression de la paroi extérieure du stent contre la paroi intérieure du vaisseau sanguin dans lequel il est inséré.

On comprend ainsi que l'invention permet bien de résoudre l'ensemble des problèmes techniques précédemment énoncés de manière simple, sûre et fiable, utilisable à l'échelle industrielle et médicale, ainsi que de mettre en oeuvre une méthode de traitement procurant un effet technique amélioré de manière inattendue par rapport à l'ETAT DE LA TECHNIQUE antérieure.

## Revendications

1. Dispositif médical implantable (10) à implanter à un lieu d'implantation, comprenant au moins un agent thérapeutiquement actif ou médicament (36), notamment hydrosoluble, **caractérisé en ce qu'**il comprend des moyens (30) de réception dudit agent actif ou médicament, sous forme de dépôt solide, le dispositif médical implantable (10) comprend une surface interne (33) et une surface externe (31), et lesdits moyens (30) pour recevoir l'agent actif ou médicament sont localisés au moins en partie sur au moins une partie de la surface externe (31) qui comprend une sculpture de surface comprenant la formation de réservoirs définissant un volume de réservoir individuel prédéterminé et permettant d'embarquer un volume ou une masse prédéterminé(e) d'agent(s) actif(s) ou médicament(s) ; et au moins une couche (38) de protection/rétention biocompatible et biodégradable permettant de réaliser la protection de l'agent actif ou médicament (36) jusqu'à son lieu d'implantation, ladite couche de protection biocompatible et biodégradable (38) comprenant au moins un agent filmogène biocompatible et biodégradable, et au moins un agent hydrophobe, de préférence biocompatible, permettant de contrôler la vitesse de désagrégation de la couche de protection/rétention.

2. Dispositif selon la revendication 1, **caractérisé en ce que** l'agent filmogène biocompatible et biodégradable est choisi parmi un polyalkylèneglycol, une polyvinylpyrrolidone, ou leurs mélanges en toutes proportions.

3. Dispositif selon la revendication 2, **caractérisé en ce que** le polyalkylèneglycol comprend ou est constitué de polyéthylèneglycol.

4. Dispositif selon l'une des revendications 1 à 3, **caractérisé en ce que** l'agent hydrophobe comprend de la dexaméthasone ou un dérivé de la dexaméthasone, tel que la dexaméthasone, la dexaméthasone phosphate et la dexaméthasone acétate.

5. Dispositif selon l'une des revendications 1 à 4, **caractérisé en ce que** le rapport relatif en poids entre l'agent filmogène biocompatible et biodégradable et l'agent hydrophobe varie de 60 % à 30 % d'agent filmogène pour 40 % à 70 % en poids d'agent hydrophobe dans la couche de protection/rétention.

6. Dispositif selon l'une des revendications 1 à 5, **caractérisé en ce que** la couche de protection/rétention comprend de 0,1 à 100 µg de dexaméthasone par mm2 de surface de couche de protection/rétention.

7. Dispositif selon l'une des revendications 1 à 6, **caractérisé en ce que** les formations de réservoirs peuvent être choisies parmi le groupe comprenant des incisions, canaux, puits ou cavités avec fonds fermés de forme concave ou non-concave, qui peuvent être de taille micrométrique, notamment comprise entre environ 1 µm et environ 600 µm, en particulier entre 10 et 150 µm, en taille moyenne, ledit(lesdits) agent(s) actif(s) ou médicament(s) étant déposé(s) à l'intérieur desdites microréservoirs sous forme d'au moins une couche de dépôt solide.

8. Dispositif selon l'une les revendications 1 à 7, **caractérisé en ce que** le dispositif médical implantable (10) comprend ou est constitué d'un stent (20) ; d'une prothèse telle que prothèse orthopédique ; d'un implant, tel que implant dentaire ; ou d'un fil ou cordon chirurgical.

9. Dispositif selon l'une les revendications 1 à 8, **caractérisé en ce que** ledit(lesdits) agent(s) thérapeutiquement actif(s) ou médicament(s) (36), notamment hydrosoluble, comprend(comprennent) un(des) agent(s) de promotion de cicatrisation sous forme de dépôt solide ; en une quantité efficace pour induire une cicatrisation ou une guérison de tissus lésés au voisinage du dispositif médical implantable.

10. Dispositif selon la revendication 9, **caractérisé en ce que** l'agent de promotion de cicatrisation comprend ou consiste en un saccharide non-réducteur ou un analogue sulfaté de celui-ci, en particulier un disaccharide non réducteur ou un analogue sulfaté de celui-ci ; du saccharose ou un analogue sulfaté de celui-ci ; un monosaccharide ou un analogue sulfaté de celui-ci; du glucose ou un analogue sulfaté de celui-ci ; du fructose ou un analogue sulfaté de celui-ci.

11. Dispositif selon la revendication 10, **caractérisé en ce que** l'analogue sulfaté est sous la forme d'un sel choisi parmi un sel de sodium, un sel de potassium, ou leurs mélanges, en particulier l'agent de promotion de cicatrisation comprend ou consiste essentiellement en du saccharose octasulfate de sodium.

12. Dispositif selon l'une des revendications 1 à 11, **caractérisé en ce que** l'agent actif ou médicament, notamment hydrosoluble (36), comprend au moins un inhibiteur de facteurs de croissance, tels que les IGF, en particulier l'octréotide ; ou un agent précurseur de NO, en particulier choisi parmi la L-arginine, la L-lysine, ou leurs mélanges ; sous forme de dépôt solide.

13. Dispositif selon l'une des revendications précédentes, **caractérisé en ce qu'**il comprend au moins un agent thérapeutique additionnel, en particulier un agent anti-resténose, par exemple choisi parmi un médicament inhibant la prolifération des cellules musculaires lisses, un inhibiteur du cytosquelette et un antibiotique triène macrocyclique.

14. Dispositif selon l'une des revendications précédentes, **caractérisé en ce que** le dispositif médical implantable (10) est un stent comprenant au moins sur sa surface externe (31) une multitude de microréservoirs (30), ledit(lesdits) agent(s) actif(s) ou médicament(s) étant déposé(s) à l'intérieur desdites microréservoirs sous forme d'au moins une couche de dépôt solide.

## Patentansprüche

1. Implantierbare medizinische Vorrichtung (10), die an einem Implantationsort zu implantieren ist, umfassend mindestens einen therapeutisch aktiven Wirkstoff oder ein Medikament (36), das insbesondere wasserlöslich ist, **dadurch gekennzeichnet, dass** sie Mittel (30) für die Aufnahme des Aktivstoffes oder Medikaments in Form eines festen Depots umfasst, wobei die implantierbare medizinische Vorrichtung (10) eine Innenfläche (33) und eine Außenfläche (31) umfasst, und die Mittel (30), um den Aktivstoff oder das Medikament aufzunehmen, zumindest teilweise auf mindestens einem Teil der Außenfläche (31) angeordnet sind, die eine Oberflächenskulptur aufweist, umfassend die Bildung von Reservoirs, die ein vorbestimmtes individuelles Reservoirvolumen definieren und es ermöglichen, ein vorbestimmtes Volumen oder eine vorbestimmte Masse von Aktivstoff(en) oder Medikament(en) unterzubringen, und mindestens eine biokompatible und biologisch abbaubare Schutz-/Halteschicht (38), die es ermöglicht, den Schutz des Aktivstoffes oder Medikaments (36) bis zu seinem Implantationsort zu verwirklichen, wobei die biokompatible und biologisch abbaubare Schicht (38) mindestens einen biokompatiblen und biologisch abbaubaren filmbildenden Wirkstoff und mindestens einen, vorzugsweise biokompatiblen, hydrophoben Wirkstoff umfasst, der es ermöglicht, die Auflösungsgeschwindigkeit der Schutz-/Halteschicht zu kontrollieren.

2. Vorrichtung gemäß Anspruch 1, **dadurch gekennzeichnet, dass** der biokompatible und biologisch abbaubare filmbildende Wirkstoff unter einem Polyalkylenglykol, Polyvinylpyrrolidon oder ihren Mischungen in allen Verhältnissen ausgewählt ist.

3. Vorrichtung gemäß Anspruch 2, **dadurch gekennzeichnet, dass** das Polyalkylenglykol Polyethylenglykol umfasst oder von diesem gebildet ist.

4. Vorrichtung gemäß einem der Ansprüche 1 bis 3, **dadurch gekennzeichnet, dass** der hydrophobe Wirkstoff Dexamethason oder ein Derivat von Dexamethason, wie Dexamethason, Dexamethasonphosphat und Dexamethasonazetat, umfasst.

5. Vorrichtung gemäß einem der Ansprüche 1 bis 4, **dadurch gekennzeichnet, dass** das relative Gewichtsverhältnis zwischen dem biokompatiblen und biologisch abbaubaren filmbildenden Wirkstoff und dem hydrophoben Wirkstoff von 60 bis 30 Gew.-% an filmbildendem Wirkstoff zu 40 bis 70 Gew.-% an hydrophobem Wirkstoff in der Schutz-/Halteschicht variiert.

6. Vorrichtung gemäß einem der Ansprüche 1 bis 5, **dadurch gekennzeichnet, dass** die Schutz-/Halteschicht 0,1 bis 100 µg Dexamethason pro mm² Fläche der Schutz-/Halteschicht umfasst.

7. Vorrichtung gemäß einem der Ansprüche 1 bis 6, **dadurch gekennzeichnet, dass** die Reservoirformationen ausgewählt sein können in der Gruppe, umfassend Einschnitte, Kanäle, Schächte oder Hohlräume mit geschlossenen Böden von konkaver oder nicht konkaver Form, die Mikrometergröße haben können, insbesondere zwischen ungefähr 1 µm und ungefähr 600 µm, insbesondere zwischen 10 und 150 µm, in durchschnittlicher Größe, wobei der(die) Aktivstoff(e) oder das(die) Medikament(e) im Inneren der Mikroreservoirs in Form von mindestens einer festen Depotschicht abgelagert werden.

8. Vorrichtung gemäß einem der Ansprüche 1 bis 7, **dadurch gekennzeichnet, dass** die implantierbare medizinische Vorrichtung (10) von einem Stent (20), einer Prothese, wie einer orthopädischen Prothese, einem Implantat, wie einem Zahnimplantat, oder einem chirurgischen Faden oder Nahtmaterial gebildet ist oder solche umfasst.

9. Vorrichtung gemäß einem der Ansprüche 1 bis 8, **dadurch gekennzeichnet, dass** der(die) therapeutisch aktive(n) Wirkstoff(e) oder das(die) Medikament(e) (36), insbesondere wasserlöslich, einen Wirkstoff(e) zur Förderung der Narbenbildung in Form eines festen Depots umfasst(en), in einer Wirkmenge, um eine Narbenbildung oder eine Heilung von verletzten Geweben in der Nähe der implantierbaren medizinischen Vorrichtung zu veranlassen.

10. Vorrichtung gemäß Anspruch 9, **dadurch gekennzeichnet, dass** der Wirkstoff zur Förderung der Narbenbildung in einem nicht reduzierenden Saccharid oder einem sulfatierten analogen Mittel zu diesem besteht oder ein solches umfasst, insbesondere ein nicht reduzierendes Disaccharid oder ein sulfatiertes analoges Mittel zu diesem, Saccharose oder ein sulfatiertes analoges Mittel zu diesem, ein Monosaccharid oder ein sulfatiertes analoges Mittel zu diesem, Glukose oder ein sulfatiertes analoges Mittel zu diesem, Fruktose oder ein sulfatiertes analoges Mittel zu diesem.

11. Vorrichtung gemäß Anspruch 10, **dadurch gekennzeichnet, dass** das analoge sulfatierte Mittel in Form eines Salzes vorhanden ist, ausgewählt unter einem Natriumsalz, einem Kaliumsalz oder ihren Mischungen, wobei der Wirkstoff zur Förderung der Narbenbildung im Wesentlichen aus Natrium-Oktasulfat-Saccharose besteht oder diese umfasst.

12. Vorrichtung gemäß einem der Ansprüche 1 bis 11, **dadurch gekennzeichnet, dass** der Aktivstoff oder das Medikament, insbesondere wasserlöslich (36), mindestens einen Wachstumsfaktor-Inhibitor, wie die IGF, insbesondere Octreotid, oder einen NO-Vorläuferwirkstoff, insbesondere ausgewählt unter L-Arginin, L-Lysin oder ihren Mischungen, in Form eines festen Depots umfasst.

13. Vorrichtung gemäß einem der vorhergehenden Ansprüche, **dadurch gekennzeichnet, dass** sie mindestens einen zusätzlichen therapeutischen Wirkstoff, insbesondere einen Restenose hemmenden Wirkstoff, umfasst, der unter einem Medikament, das die Vermehrung der glatten Muskelzellen hemmt, einem Inhibitor des Zytoskeletts und einem makrozyklischen Trien-Antibiotikum ausgewählt ist.

14. Vorrichtung gemäß einem der vorhergehenden Ansprüche, **dadurch gekennzeichnet, dass** die implantierbare medizinische Vorrichtung (10) ein Stent ist, umfassend mindestens auf seiner Außenseite (31) eine Vielzahl von Mikroreservoirs (30), wobei der(die) Aktivstoff(e) oder das(die) Medikament(e) im Inneren der Mikroreservoirs in Form mindestens einer festen Depotschicht aufgebracht werden.

## Claims

1. An implantable medical device (10) to be implanted at a site of implantation, comprising at least one therapeutically active agent or medicament (36), in particular a water-soluble one, **characterized in that** it comprises means (30) for receiving said active agent or medicament, in the form of a solid deposit, the implantable medical device (10) comprises an internal surface (33) and an external surface (31), and said means (30) for receiving the active agent or medicament are at least in part located on at least a part of the external surface (31) which comprises surface sculpturing comprising the formation of reservoirs defining a predetermined individual reservoir volume and enabling a predetermined volume or mass of active agent(s) or medicament(s) to be loaded; and at least one biocompatible and biodegradable protecting/retaining layer (38) for protecting the active agent or medicament (36) until it is at its site of implantation, said biocompatible and biodegradable protecting/retaining layer (38) comprising at least one biocompatible and biodegradable film-forming agent and at least one hydrophobic, preferably biocompatible, agent for controlling the disintegration rate of the protecting/retaining layer.

2. The device as claimed in claim 1, **characterized in that** the biocompatible and biodegradable film-forming agent is selected from a polyalkylene glycol, a polyvinylpyrrolidone and mixtures thereof in any proportions.

3. The device as claimed in claim 2, **characterized in that** the polyalkylene glycol comprises or consists of polyethylene glycol.

4. The device as claimed in one of claims 1 to 3, **characterized in that** the hydrophobic agent comprises dexamethasone or a dexamethasone derivative, such as dexamethasone, dexamethasone phosphate and dexamethasone acetate.

5. The device as claimed in one of claims 1 to 4, **characterized in that** the relative ratio by weight of the biocompatible and biodegradable film-forming agent to the hydrophobic agent varies between 60 % and 30% of film-forming agent for 40% to 70% by weight of hydrophobic agent in the protecting/retaining layer.

6. The device as claimed in one of claims 1 to 5, **characterized in that** the protecting/retaining layer comprises from 0.1 to 100 µg of dexamethasone per mm² of protecting/retaining layer surface.

7. The device as claimed in one of claims 1 to 6, **characterized in that** the reservoir formations can be selected from the group comprising incisions, channels, wells or cavities with closed bottoms of concave or nonconcave shape which may be of micrometric size, notably between approximately 1 µm and approximately 600 µm, in particular between 10 and 150 µm, with respect to average size, said active agent(s) or medicament(s) being located inside the microreservoirs in the form of at least one layer of solid deposit.

8. The device as claimed in one of claims 1 to 7, **characterized in that** the implantable medical device (10) comprises or consists of a stent (20); of a prosthesis such as an orthopedic prosthesis; of an implant such as a dental implant; or of a surgical thread or cord.

9. The device as claimed in one of claims 1 to 8, **characterized in that** said therapeutically active agent(s) or medicament(s) (36), in particular water-soluble one(s), comprise(s) one or more cicatrization-promoting agent(s) in the form of a solid deposit; in an effective amount for inducing cicatrization or healing of injured tissues in the vicinity of the implantable medical device.

10. The device as claimed in claim 9, **characterized in that** the cicatrization-promoting agent comprises or consists of a nonreducing saccharide or a sulfated analog thereof, in particular a nonreducing disaccharide or a sulfated analog thereof; of sucrose or a sulfated analog thereof, of a monosaccharide or a sulfated analog thereof; of glucose or a sulfated analog thereof; of fructose or a sulfated analog thereof.

11. The device as claimed in claim 10, **characterized in that** in the sulfated analog is in the form of a salt selected from a sodium salt, a potassium salt and mixtures thereof, in particular the cicatrization-promoting agent comprises or essentially consists of sodium sucrose octasulfate.

12. The device as claimed in one of claims 1 to 11, **characterized in that** the active agent or medicament, in particular a water-soluble one, (36) comprises at least one growth inhibitor of growth factors, such as IGFs, in particular octreotide; or an NO precursor agent, in particular selected from L-arginine, L-lysine and mixtures thereof; in the form of a solid deposit.

13. The device as claimed in one of the preceding claims, **characterized in that** it comprises at least one additional therapeutic agent, in particular an anti-restenosis agent, for example selected from a medicament inhibiting smooth muscle cell proliferation, a cytoskeletal inhibitor, and a macrocyclic triene antibiotic.

14. The device as claimed in one of the preceding claims, **characterized in that** the medical implantable device (10) is a stent comprising at least one its external surface (31) a multitude of microreservoirs (30), said active agent(s) or medicament(s) being located inside said microreservoirs in the form of at least one layer of solid deposit.
